# EUROPEAN PATENT SPECIFICATION

(11) **EP 4 046 614 B1**
(45) Date of publication and mention of the grant of the patent: **25.03.2026**
(21) Application number: 20890640.4
(22) Date of filing: 18.11.2020
(51) Int. Cl.: A61F 13/15, A61F 13/49, A61F 13/511

(54) **DISPOSABLE DIAPER**
WEGWERFWINDEL
COUCHE JETABLE

(30) Priority: 20.11.2019 JP 2019209786
(43) Date of publication of application: 24.08.2022
(73) Proprietor: Daio Paper Corporation, Ehime 799-0492 (JP)
(72) Inventor: FURUKAWA, Masashi, Shikokuchuo-shi, Ehime 799-0431 (JP)
(74) Representative: Meissner Bolte Partnerschaft mbB
(86) International application number: PCT/JP2020/043053
(87) International publication number: WO 2021/100774

(56) References cited:
- EP-A1- 1 671 609
- WO-A1-2004/050000
- WO-A1-2018/100650
- WO-A1-2019/005666
- JP-A- 2004 255 164
- JP-A- 2013 066 598
- JP-A- 2018 102 836
- US-A1- 2019 000 688

## Description

### Technical Field

The present invention relates to a disposable diaper, particularly to a disposable diaper that moisturizes the skin of a wearer and improves fitting to the wearer.

### Background Art

In order to suppress adhesion of feces excreted in a disposable diaper to the skin of a wearer, a technique of applying a lotion agent to a dorsal side portion of a body-side surface of a top sheet is known. (Patent Literature 1)
Patent Literature 2 relates to absorbent articles, such as diapers and sanitary napkins, and topsheets useful for such articles.
Patent Literature 3 relates to an absorbent article including an outer cover member, a liner, and an absorbent body structure disposed between the cover member and the liner.
Patent Literature 4 relates to an absorbent article having a longitudinal axis extending in a longitudinal direction and a transversal axis extending in a transversal direction perpendicular to the longitudinal direction
Patent Literature 5 relates to absorbent articles such as diapers, sanitary napkins, incontinence pads, panty liners, and vaginal discharge sheets, which contain skin care agents.
Patent Literature 6 relates to a method for manufacturing absorbent articles such as disposable diapers and sanitary napkins.

### Citation List

### Patent Literature

Patent Literature 1: JP 2010-75733 A
Patent Literature 2: EP 1 671 609 A1
Patent Literature 3: WO 2004/050000 Al
Patent Literature 4: WO 2019/005666 A1
Patent Literature 5: JP2004-255164
Patent Literature 6: JP2013-66598

### Summary of Invention

### Technical Problem

However, in the technique of Patent Literature 1, there is a possibility that an adhesive force of an end flap portion on a dorsal side of a disposable diaper decreases and a top sheet is rolled up toward a body surface side, and there is a possibility that an adhesive force of a stretchable sheet disposed in the end flap portion on the dorsal side decreases and a stretchable member cannot achieve elasticity.

Therefore, an object of the present invention is to provide a disposable diaper capable of moisturizing the skin of a wearer and suitably maintaining an adhesive force of an end flap portion.

### Solution to Problem

A means solving the above problem is as follows.

A first means is a disposable diaper including: an absorber disposed between a liquid pervious top sheet and a liquid impervious back sheet; an end flap portion formed on each side of the absorber in a front-back direction; and a lotion portion extending in the front-back direction at a predetermined interval in a width direction on a body-side surface of the top sheet, wherein in the front-back direction, the lotion portion is disposed in an intermediate portion between a ventral side portion accounting for 20 to 45% of a front-back direction length of the top sheet from a ventral side end of the top sheet and a dorsal side portion accounting for 10 to 35% of the front-back direction length of the top sheet from a dorsal side end of the top sheet, and
the lotion portion is disposed in each of the ventral side portion and the dorsal side portion, and a second application basis weight of a lotion agent forming the lotion portion disposed in each of the ventral side portion and the dorsal side portion is smaller than a first application basis weight of the lotion agent forming the lotion portion disposed in the intermediate portion.

As defined in the invention according to claim 1, the lotion portion is disposed in each of the ventral side portion and the dorsal side portion, and a second application basis weight of a lotion agent forming the lotion portion disposed in each of the ventral side portion and the dorsal side portion is smaller than a first application basis weight of the lotion agent forming the lotion portion disposed in the intermediate portion.

A second means is the disposable diaper recited in the first means, wherein the first application basis weight is 5 to 15 g/m², and the second application basis weight is 2 to 5 g/m².

A third means is the disposable diaper recited in any one of the first to second means, wherein a rectangular stretchable sheet that stretches and contracts in the width direction is disposed between the top sheet and the back sheet forming the end flap portion on a dorsal side, the stretchable sheet is formed of an inner sheet facing the top sheet, an outer sheet facing the back sheet, and a stretchable member that stretches and contracts in the width direction between the inner sheet and the outer sheet at a predetermined interval in the front-back direction, the stretchable member is fixed to an opposite body-side surface of the inner sheet and a body-side surface of the outer sheet via an adhesive portion formed at a predetermined interval in the width direction, and in plan view, a first interval of the lotion portion in the width direction is wider than a second interval between the adhesive portion and the adhesive portion adjacent thereto in the width direction.

A fourth means is the disposable diaper recited in any one of the first to third means, wherein the lotion agent is water-soluble glycerin.

### Advantageous Effects of Invention

According to the first means, an absorber is disposed between a liquid pervious top sheet and a liquid impervious back sheet, an end flap portion is formed on each side of the absorber in a front-back direction, a lotion portion extending in the front-back direction at a predetermined interval in a width direction is formed on a body-side surface of the top sheet, and in the front-back direction, the lotion portion is disposed in an intermediate portion between a ventral side portion accounting for 20 to 45% of a front-back direction length of the top sheet from a ventral side end of the top sheet and a dorsal side portion accounting for 10 to 35% of the front-back direction length of the top sheet from a dorsal side end of the top sheet. Therefore, the skin of a wearer, particularly a crotch portion can be moisturized to enhance a texture and a skin care effect. In addition, a decrease in adhesive force of the end flap portion can be suppressed to prevent the top sheet from being rolled **up.**

According to the first means, the lotion portion is disposed in each of the ventral side portion and the dorsal side portion, and the second application basis weight of a lotion agent forming the lotion portion disposed on each of the ventral side and the dorsal side is smaller than the first application basis weight of the lotion agent forming the lotion portion disposed in the intermediate portion. Therefore, the ventral side portion and the dorsal side portion of a wearer can be moisturized to further enhance a texture and a skin care effect.

According to the second means, in addition to the effect of the first means, the first application basis weight is 5 to 15 g/m², and the second application basis weight is 2 to 5 g/m². Therefore, the lotion agent or the like forming the lotion portion can be prevented from being excessively transferred to a wearer.

According to the third means, in addition to the effect of any one of the first to second means, a rectangular stretchable sheet that stretches and contracts in the width direction is disposed between the top sheet and the back sheet forming the end flap portion on a dorsal side, the stretchable sheet is formed of an inner sheet facing the top sheet, an outer sheet facing the back sheet, and a stretchable member that stretches and contracts in the width direction between the inner sheet and the outer sheet at a predetermined interval in the front-back direction, the stretchable member is fixed to an opposite body-side surface of the inner sheet and a body-side surface of the outer sheet via an adhesive portion formed at a predetermined interval in the width direction, and in plan view, a first interval of the lotion portion in the width direction is wider than a second interval between the adhesive portion and the adhesive portion adjacent thereto in the width direction. Therefore, the lotion portion can be uniformly disposed on the top sheet. In addition, when the lotion portion is disposed in the dorsal side portion, a pleat formed on the end flap portion on the dorsal side is large, and fitting can be further enhanced. Furthermore, a decrease in adhesive force of the stretchable sheet can be suppressed, and a wrinkle can be formed in the end flap portion on the dorsal side to maintain high fitting.

According to the fourth means, in addition to the effect of any one of the first to third means, the lotion agent is water-soluble glycerin. Therefore, moisture in the surrounding air can be drawn to further moisturize the skin of a wearer.

### Brief Description of Drawings

Fig. 1 is a plan view of a body side of a disposable diaper.
Fig. 2 is a plan view of an opposite body side of the disposable diaper.
Fig. 3 is a cross-sectional view taken along line X1-X1 of Fig. 1.
Fig. 4 is a plan view of a stretchable sheet.
Fig. 5 is a plan view of a lotion application form in a first embodiment.
Fig. 6 is a cross-sectional view taken along line X2-X2 of Fig. 5 for describing an application form of the lotion.
Fig. 7 is a plan view of a dorsal side end flap portion for describing an application form of the lotion.
Fig. 8 is a plan view of a lotion application form in a second embodiment.
Fig. 9 is a cross-sectional view taken along line X2-X2 of Fig. 8 for describing an application form of the lotion.
Fig. 10 is a plan view of a dorsal side end flap portion for describing an application form of the lotion.
Fig. 11 is an explanatory diagram of a form of a pleat of a stretchable sheet before a lotion is applied thereto.
Fig. 12 is an explanatory diagram of a form of a pleat of a stretchable sheet after a lotion is applied thereto.
Fig. 13 is an explanatory diagram of a moroccan pattern.

### Description of Embodiments

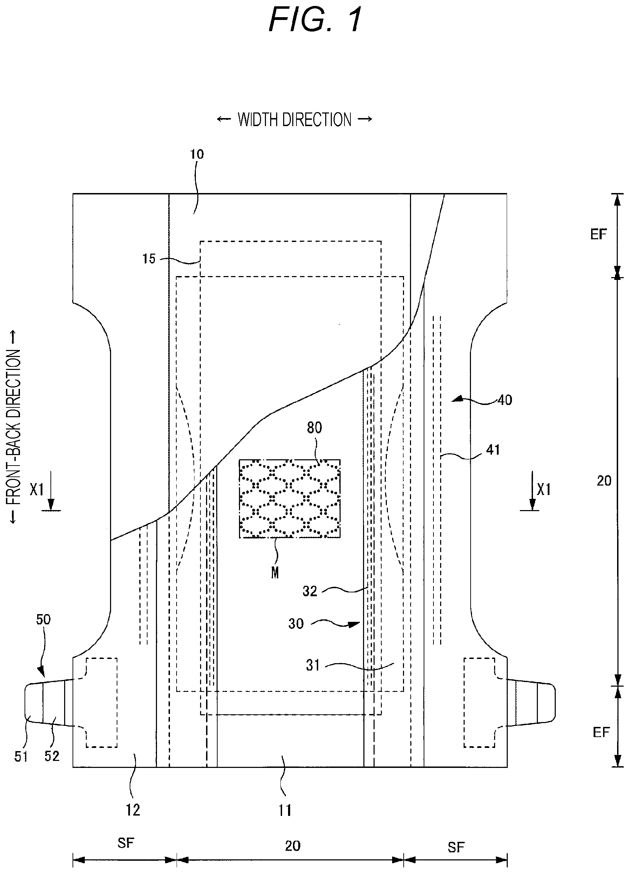

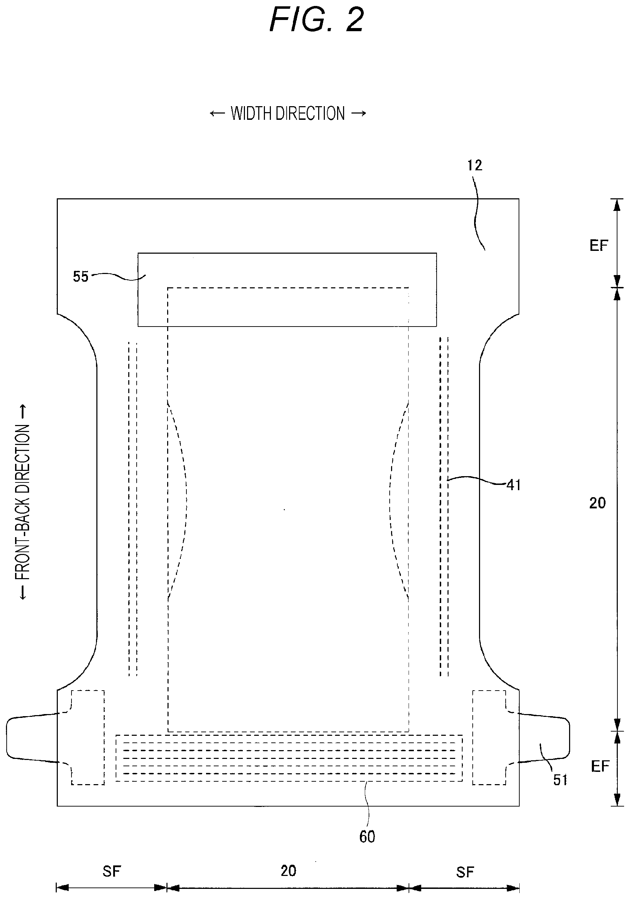

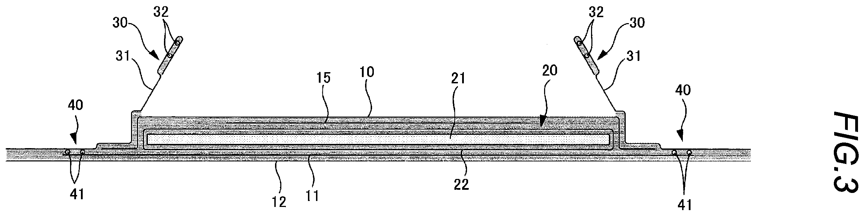

As illustrated in Figs. 1 to 3, a disposable diaper includes a liquid pervious top sheet 10 disposed on a body side, a liquid impervious back sheet 11 disposed on an opposite body side, and an absorbent element 20 disposed between the top sheet 10 and the back sheet 11. The absorbent element 20 includes an absorber 21 and a wrapping sheet 22 covering the absorber 21. Note that a plurality of apertures 80 is formed in the top sheet 10, and imaginary lines 81 connecting adjacent apertures 80 to each other are formed in a moroccan pattern M.

An outer sheet 12 is disposed on an opposite body side of the back sheet 11. It is preferable to dispose an intermediate sheet 15 that moves excrement that has passed through the top sheet 10 to the absorbent element 20 between the top sheet 10 and the absorbent element 20 to prevent returning of the excrement.

A three-dimensional gather 30 that prevents leakage of excrement to the outside is formed at a predetermined interval on each side of the absorbent element 20 in a width direction. The three-dimensional gather 30 includes a gather sheet 31 substantially continuous in the width direction and an elongated elastically stretchable member 32 fixed to the gather sheet 31 in a stretched state in a front-back direction.

A planar gather 40 that prevents leakage of ureteral to the outside is formed at a predetermined interval outside a base of the three-dimensional gather 30. The planar gather 40 includes an elongated elastically stretchable member 41 fixed between the back sheet 11 and the gather sheets 31 in a stretched state in the front-back direction.

An end flap portion EF is formed on each side of the absorbent element 20 in the front-back direction, and a side flap portion SF is formed on each side of the absorbent element 20 in the width direction.

A fastening tape 50 is disposed on each side of a dorsal side portion of the side flap portion SF in the width direction. The fastening tape 50 includes a base material 51 fixed to the side flap portion SF and an engagement portion 52 disposed on a body side of the base material 51.

A rectangular target sheet 55 to be engaged with the engagement portion 52 of the fastening tape 50 and extending in the width direction at a predetermined interval in the front-back direction is disposed on a ventral side portion on an opposite body side of the outer sheet 12.

A stretchable sheet 60 described later is disposed between the top sheet 10 and the back sheet 11 forming the end flap portion EF on a dorsal side.

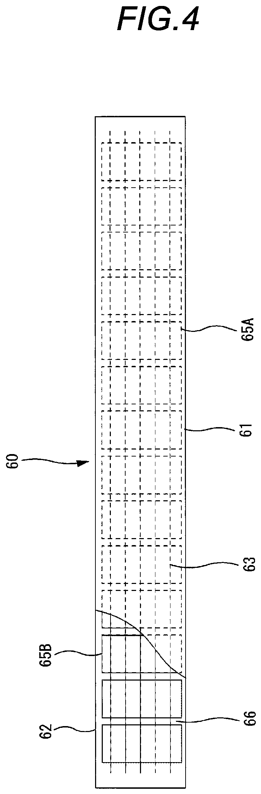

As illustrated in Fig. 4, the stretchable sheet 60 includes an inner sheet 61 facing the top sheet 10, an outer sheet 62 facing the back sheet 11, and an elongated stretchable member 63 fixed between the inner sheet 61 and the outer sheet 62 in a stretched state in the width direction.

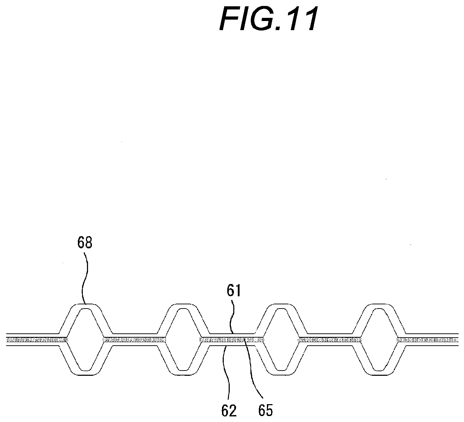

The stretchable member 63 is fixed to a rectangular first adhesive portion 65A formed on an opposite body-side surface of the inner sheet 61 at a predetermined interval in the width direction and a second adhesive portion 65B formed on a body surface side of the outer sheet 62 at a predetermined interval in the width direction. As a result, as illustrated in Fig. 11, a substantially mountain-shaped wrinkle 68 can be formed on a body-side surface and an opposite body-side surface of the stretchable sheet 60 to fit the disposable diaper to a dorsal side waist portion of a wearer. The first adhesive portion 65A and the second adhesive portion 65B can be formed by applying a hot melt adhesive. Note that in the present specification, the first adhesive portion 65A and the second adhesive portion 65B are collectively referred to as an adhesive portion 65, and a site between the adhesive portion 65 and the adhesive portion 65 adjacent thereto is referred to as a non-adhesive portion 66.

Instead of forming the adhesive portion 65 on each of the inner sheet 61 and the outer sheet 62, the adhesive portion 65 can be formed on an outer peripheral portion of the stretchable member 63 at a predetermined interval corresponding to the above-described non-adhesive portion 66 in the width direction to fix the inner sheet 61 and the outer sheet 62 to the stretchable member 63.

### <First Embodiment>

Next, a form of a lotion portion 70 of a first embodiment will be described.

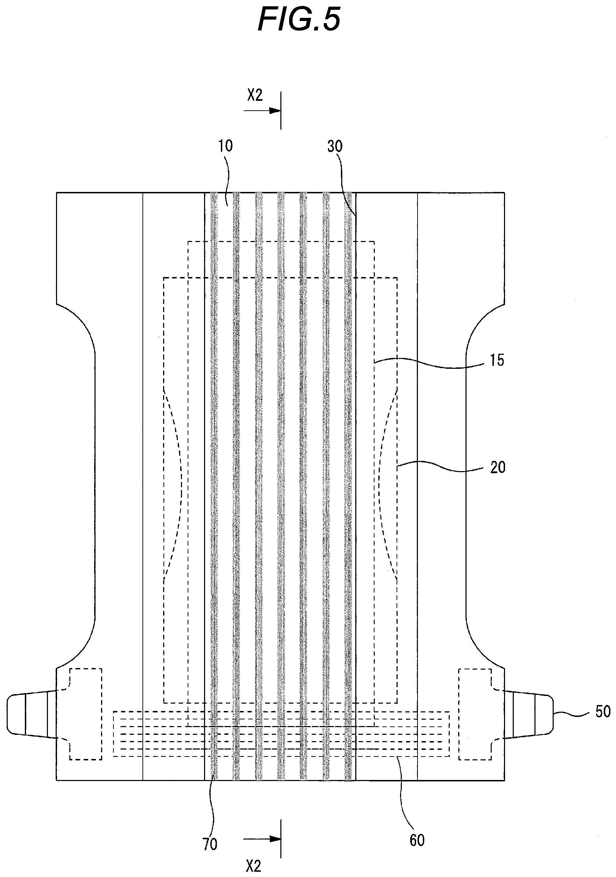

As illustrated in Fig. 5, at a site between the three-dimensional gathers 30 on a body-side surface of the top sheet 10, the lotion portion 70 extending in the front-back direction is disposed at a predetermined interval in the width direction. Note that also to a site between the lotion portion 70 and the lotion portion on the body-side surface of the top sheet 10, a lotion agent can be applied in an amount smaller than that in the lotion portion 70. This makes a contact portion between the disposable diaper and a wearer smooth, for example, brings a ventral side end flap portion EF and a ventral side waist portion of the wearer into smooth contact with each other, and brings a dorsal side end flap portion EF and a dorsal side waist portion of the wearer into smooth contact with each other, which can enhance a texture and suppress a skin damage of the wearer to enhance a skin care effect. Note that the lotion portion 70 can be formed by applying and transferring a lotion agent.

As the lotion agent, water-soluble glycerin can be used, but a lotion agent formed by diluting glycerin with a predetermined amount of aqueous solution is preferable. This makes it possible to draw moisture in the surrounding air to moisturize the skin of a wearer. In addition, hyaluronic acid, collagen, and ceramide can be added to glycerin to enhance a moisturizing power of the lotion agent, and an optimal lotion can be designed and used according to a use time and a use place of the disposable diaper. Furthermore, oily petrolatum or the like can be added to impart a barrier function to the skin.

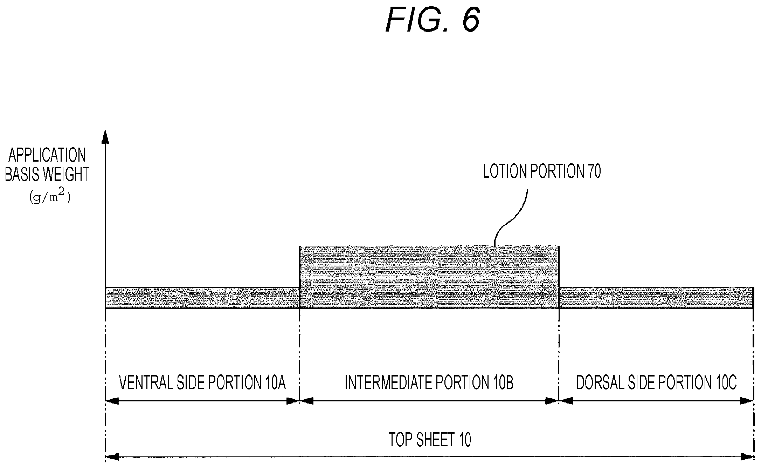

As illustrated in Fig. 6, an application basis weight ("second application basis weight" in the claims) of a lotion agent forming the lotion portion 70 disposed in each of a ventral side portion 10A and a dorsal side portion 10C of the top sheet 10 in the front-back direction is smaller than an application basis weight ("first application basis weight" in the claims) of the lotion agent forming the lotion portion 70 disposed in an intermediate portion 10B. As a result, it is possible to suppress a decrease in adhesive strength between the top sheet 10 and the back sheet 11 forming the end flap portion EF.

In the present specification, the ventral side portion 10A of the top sheet 10 refers to a site of 20 to 45%, preferably 25 to 40%, of the front-back direction length of the top sheet 10 from a ventral side end of the top sheet 10 toward the dorsal side. As a result, a decrease in adhesive strength can be suppressed in the entire region of the top sheet 10 and the back sheet 11 forming the end flap portion EF on the ventral side.

The intermediate portion 10B of the top sheet 10 refers to a site of 20 to 70%, preferably 30 to 55%, of the front-back direction length of the top sheet 10 from a dorsal side end of the ventral side portion 10A toward the dorsal side.

Furthermore, the dorsal side portion 10C of the top sheet 10 in the front-back direction refers to a site of 10 to 35%, preferably 20 to 30%, of the front-back direction length of the top sheet 10 from a dorsal side end of the intermediate portion 10B toward the dorsal side, that is, a site from the dorsal side end of the intermediate portion 10B to a dorsal side end of the top sheet 10. As a result, a decrease in adhesive strength can be suppressed in the entire region of the top sheet 10 and the back sheet 11 forming the end flap portion EF on the dorsal side. Note that Fig. 6 illustrates a form in which the length of the ventral side portion 10A of the top sheet 10 is formed to be 30% of the front-back direction length of the top sheet 10, the length of the intermediate portion 10B of the top sheet 10 is formed to be 40% of the front-back direction length of the top sheet 10, and the length of the dorsal side portion 10C of the top sheet 10 is formed to be 30% of the front-back direction length of the top sheet 10.

The application basis weight of the lotion agent forming the lotion portion 70 disposed on a body surface side of the ventral side portion 10A of the top sheet 10 is preferably 2 to 5 g/m². When the application basis weight is less than 2 g/m², textures of the ventral side portion 10A of the top sheet 10 and the ventral waist portion of a wearer may be deteriorated, and a skin care effect may be also deteriorated. Meanwhile, when the application basis weight is more than 5 g/m², an adhesive strength between the top sheet 10 and the back sheet 11 forming the end flap portion EF on the ventral side may decrease.

The application basis weight of the lotion agent forming the lotion portion 70 disposed on a body surface side of the intermediate portion 10B of the top sheet 10 is preferably 5 to 15 g/m². When the application basis weight is less than 5 g/m², textures of the intermediate portion 10B of the top sheet 10 and the crotch portion of a wearer may be deteriorated, and a skin care effect may be also deteriorated. Meanwhile, when the application basis weight is more than 15 g/m², the lotion agent may be transferred to the crotch portion of a wearer to excessively moisturize the crotch portion of the wearer.

The application basis weight of the dorsal side portion 10C of the top sheet 10 is preferably 2 to 5 g/m². When the application basis weight less than 2 g/m², textures of the dorsal side portion 10C and a dorsal side waist portion of a wearer may be deteriorated, and a skin care effect may be also deteriorated. Meanwhile, when the application basis weight is more than 5 g/m², an adhesive strength between the top sheet 10 and the back sheet 11 forming the end flap portion EF on the dorsal side may decrease. In addition, an adhesive strength between the stretchable member 63 of the stretchable sheet 60 disposed in the end flap portion EF on the dorsal side and each of the inner sheet 61 and the outer sheet 62 may decrease.

An area ratio of the lotion portion 70, that is, a ratio of the area of the lotion portion 70 to the area of a site between the three-dimensional gathers 30 on a body-side surface of the top sheet 10 is preferably 20 to 70%. When the area ratio is less than 20%, it may be difficult to form the lotion portion 70 continuous in the front-back direction at a predetermined interval in the width direction. When the area ratio is more than 70%, the skin of a wearer may be excessively moisturized.

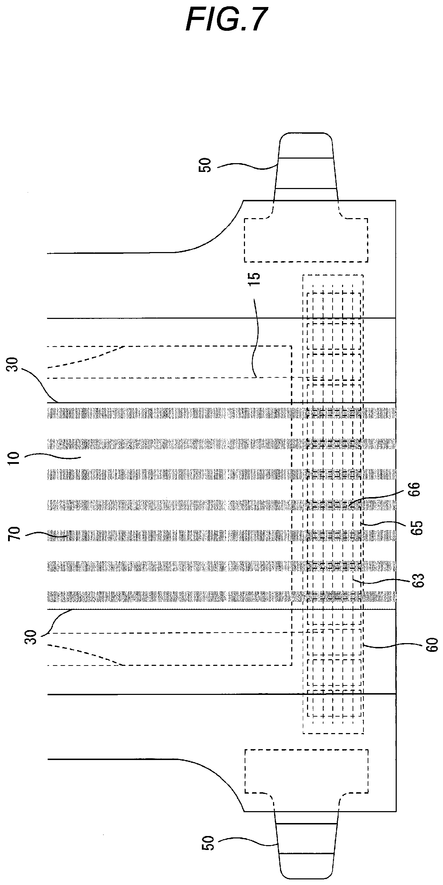

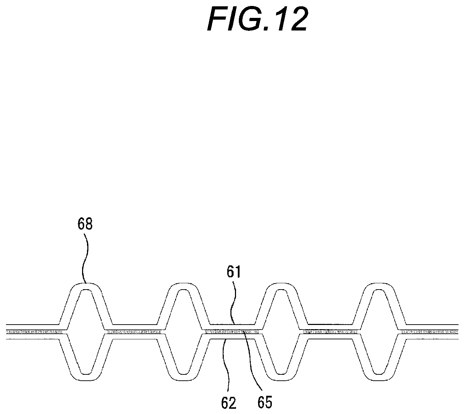

As illustrated in Fig. 7, the lotion portion 70 extends in the front-back direction at a site corresponding to the non-adhesive portion 66 extending in the front-back direction in the stretchable sheet 60 disposed in the end flap portion EF on the dorsal side. An interval ("first interval" in the claims) of the lotion portion 70 in the width direction is formed to be wider than an interval ("second interval" in the claims) between the adhesive portion 65 and the adhesive portion 65 adjacent thereto in the width direction. That is, a left side portion of the lotion portion 70 in the width direction overlaps a right side portion of the adhesive portion 65 formed on the left side of the non-adhesive portion 66, and a right side portion of the lotion portion 70 in the width direction overlaps a left side portion of the adhesive portion 65 formed on the right side of the non-adhesive portion 66. As a result, an adhesive force between the right side portion of the adhesive portion 65 overlapping the left side portion of the lotion portion 70 in the width direction and the left side portion of the adhesive portion 65 overlapping the right side portion of the lotion portion 70 in the width direction is weakened, and as illustrated in Fig. 12, the wrinkle 68 formed on a body-side surface of the stretchable sheet 60 is extended more to a body side, and the disposable diaper can be thereby fitted to the dorsal side waist portion of a wearer more sufficiently.

### <Second Embodiment>

Next, a form of a lotion portion 70 of a second embodiment will be described. The same members as those of the lotion portion 70 of the first embodiment are denoted by the same reference characters, and description thereof will be omitted.

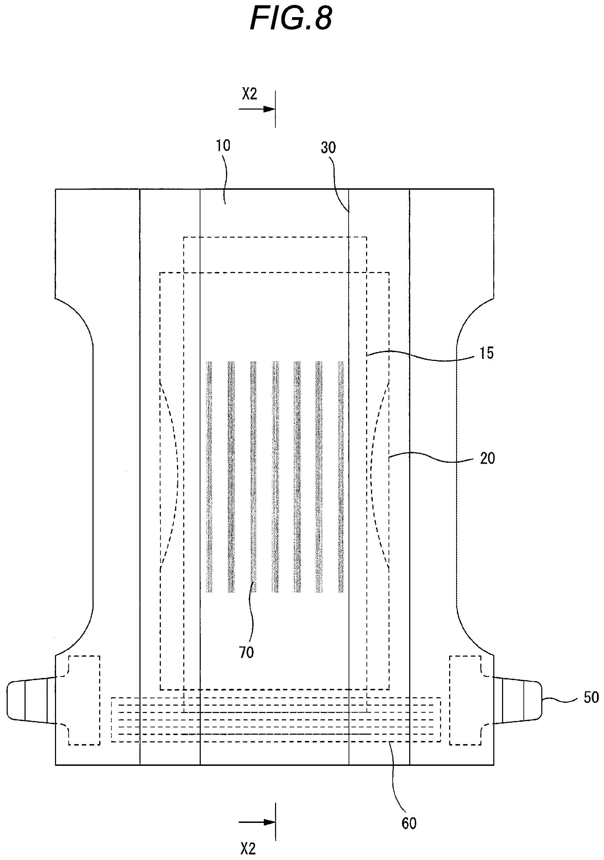

As illustrated in Fig. 8, at a site between the three-dimensional gathers 30 on a body-side surface of the intermediate portion 10B of the top sheet 10, the lotion portion 70 extending in the front-back direction with a predetermined length is formed at a predetermined interval in the width direction. In addition, a ventral side end of the lotion portion 70 is located on a dorsal side of a site corresponding to a ventral side end of the absorbent element 20 in the top sheet 10, and a dorsal side end of the lotion portion 70 is located on a ventral side of a site facing a dorsal side end of the absorbent element 20 in the top sheet 10. This makes a contact portion between the disposable diaper and a wearer smooth, for example, brings the disposable diaper and a crotch portion of the wearer into smooth contact with each other, which can enhance a texture and suppress a skin damage of the wearer to enhance a skin care effect. In addition, a decrease in adhesive strength can be suppressed in the entire region of the top sheet 10 and the back sheet 11 forming the end flap portion EF. Furthermore, a decrease in adhesive strength between the stretchable member 63 of the stretchable sheet 60 disposed in the end flap portion EF on the dorsal side and each of the inner sheet 61 and the outer sheet 62 can be prevented.

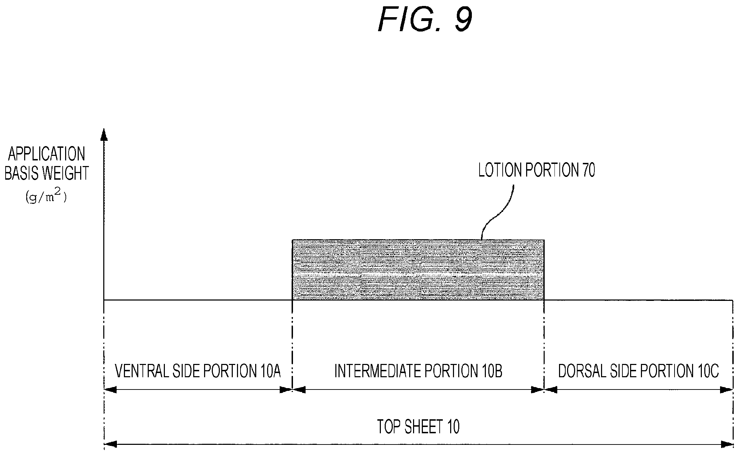

As illustrated in Fig. 9, an application basis weight of a lotion agent forming the lotion portion 70 disposed on a body surface side of the intermediate portion 10B of the top sheet 10 is preferably 5 to 15 g/m². When the application basis weight is less than 5 g/m², textures of the ventral side portion 10A of the top sheet 10 and the ventral waist portion of a wearer may be deteriorated, and a skin care effect may be also deteriorated. Meanwhile, when the application basis weight is more than 15 g/m², an adhesive strength between the top sheet 10 and the back sheet 11 forming the end flap portion EF on the ventral side may decrease. Note that Fig. 9 illustrates a form in which the length of the ventral side portion 10A of the top sheet 10 is formed to be 30% of the front-back direction length of the top sheet 10, the length of the intermediate portion 10B of the top sheet 10 is formed to be 40% of the front-back direction length of the top sheet 10, and the length of the dorsal side portion 10C of the top sheet 10 is formed to be 30% of the front-back direction length of the top sheet 10.

An area ratio of the lotion portion 70, that is, a ratio of the area of the lotion portion 70 to the area of a site between the three-dimensional gathers 30 in the intermediate portion 10B on a body-side surface of the top sheet 10 is preferably 20 to 70%. When the area ratio is less than 20%, it may be difficult to form the lotion portion 70 continuous in the front-back direction at a predetermined interval in the width direction. When the area ratio is more than 70%, the skin of a wearer may be sticky, and the wearer may feel uncomfortable.

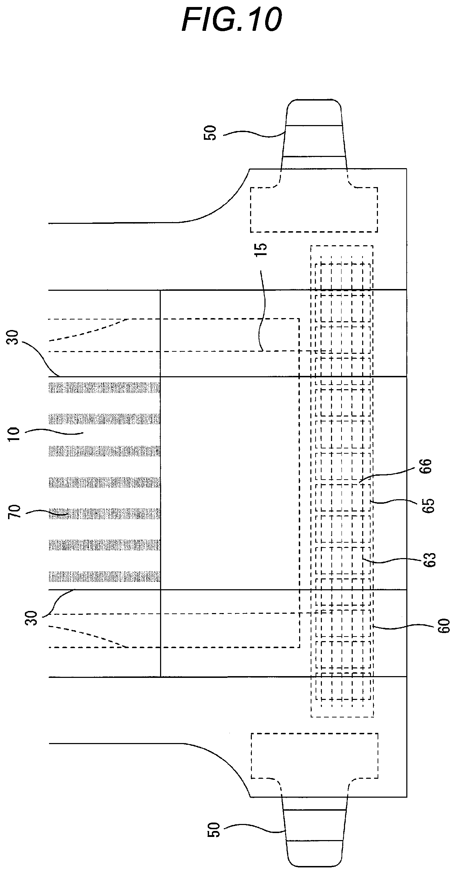

As illustrated in Fig. 10, the lotion portion 70 is located at a site corresponding to the non-adhesive portion 66 extending in the front-back direction in the stretchable sheet 60 disposed in the end flap portion EF on the dorsal side. An interval of the lotion portion 70 in the width direction is formed to be wider than an interval between the adhesive portion 65 and the adhesive portion 65 adjacent thereto in the width direction.

Next, materials and characteristic portions of the top sheet 10 and the like will be sequentially described.

### (Top sheet)

The top sheet 10 is formed of a perforate or imperforate nonwoven fabric, a porous plastic sheet, or the like. Among these materials, the nonwoven fabric is not particularly limited concerning a raw material fiber thereof. Examples thereof include a synthetic fiber such as a polyolefin-based fiber including polyethylene and polypropylene, a polyester-based fiber, or a polyamide-based fiber, a regenerated fiber such as rayon or cupra, a natural fiber such as cotton, and a mixed fiber and a composite fiber in which two or more kinds of these fibers are used. Furthermore, the nonwoven fabric may be manufactured by any processing. Examples of a processing method include known methods such as a spunlace method, a spunbond method, a thermal bond method, a melt blown method, a needle punching method, an air through method, and a point bond method. For example, if softness and drapeability are demanded, a spunlace method is a preferable processing method. If bulkiness and softness are demanded, a thermal bond method is a preferable processing method.

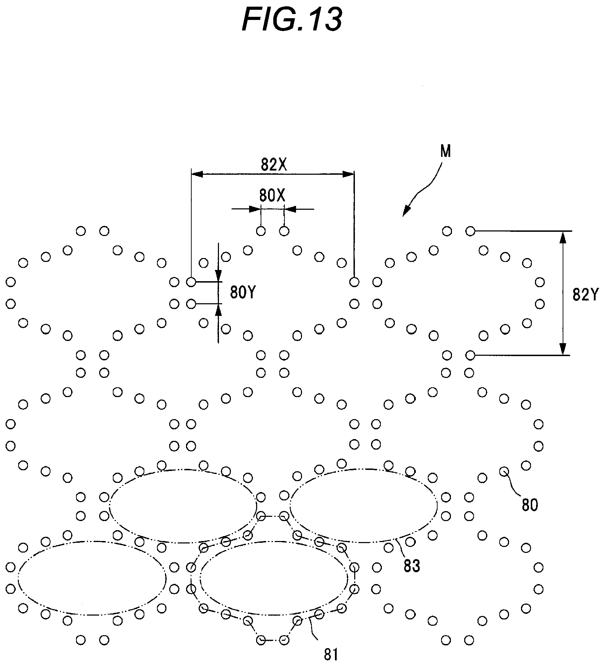

As illustrated in Fig. 13, the plurality of apertures 80 is formed in the top sheet 10. The diameter of the aperture 80 is 0.2 to 1.5 mm, and preferably 0.3 to 1.0 mm. An interval 80X between the apertures 80 adjacent to each other in the width direction is 2.0 to 10 mm, and preferably 3.0 to 5.0 mm. An interval 80Y between the apertures 80 adjacent to each other in the front-back direction is 0.9 to 8.0 mm, and preferably 1.0 to 3.0 mm. This can enhance air permeability of the top sheet 10 to enhance a skin care effect.

The imaginary lines 81 connecting the apertures 80 adjacent to each other are formed in a substantially elliptical moroccan pattern M in which a major axis is formed in the width direction. The size of the moroccan pattern M is not particularly limited, but in consideration of the strength of the top sheet 10 or the like, an interval 82X of the moroccan pattern M in the width direction is 10 to 30 mm, and preferably 15 to 25 mm, and an interval 82Y of the moroccan pattern M in the front-back direction is 5 to 20 mm, and preferably 8 to 15 mm. Note that a non-aperture region 83 surrounded by a group of the apertures 80 is a non-deformed or hardly deformed region. However, since sites of imaginary connection lines 84 and 85 are easily deformed, the non-aperture region 83 can easily follow a change in the posture of a wearer.

### (Back sheet)

The back sheet 11 is formed of a polyolefin-based resin such as polyethylene or polypropylene, a laminated nonwoven fabric obtained by stacking a nonwoven fabric on a polyethylene sheet or the like, or a nonwoven fabric in which a waterproof film is interposed to substantially secure a liquid impervious property (in this case, the waterproof film and the nonwoven fabric form the back sheet 11) or the like. Of course, in addition to these materials, a liquid impervious and moisture permeable material that has been favorably used from a viewpoint of preventing stuffiness in recent years can be used, for example. Examples of a sheet of the liquid impervious and moisture permeable material include a microporous sheet obtained by kneading an inorganic filler in a polyolefin-based resin such as polyethylene or polypropylene, molding a sheet, and then stretching the sheet in a monoaxial or biaxial direction. Furthermore, a nonwoven fabric using a micro denier fiber, and a sheet that has become liquid impervious without using a waterproof film by a method for reinforcing leakproofness by reducing a space between fibers by applying heat and pressure or a method for applying a super absorbent resin, a hydrophobic resin, or a water repellent agent, can be used as the back sheet 11.

### (Outer sheet)

The outer sheet 12 is a portion for supporting the absorbent element 20 and being worn by a wearer. The outer sheet 12 has an hourglass shape in which central portions of both side portions in the front-back direction are narrowed, and the central portions surround the legs of a wearer.

The outer sheet 12 is preferably formed of a nonwoven fabric. The type of the nonwoven fabric is not particularly limited. Examples thereof as a material fiber include a synthetic fiber such as a polyolefin-based fiber including polyethylene and polypropylene, a polyester-based fiber, or a polyamide-based fiber, a regenerated fiber such as rayon or cupra, and a natural fiber such as cotton. Examples of a processing method include a spunlace method, a spunbond method, a thermal bond method, an air through method, and a needle punching method. However, a long-fiber nonwoven fabric such as a spunbonded nonwoven fabric, an SMS nonwoven fabric, or an SMMS nonwoven fabric is suitable from a viewpoint of achieving both texture and strength. A nonwoven fabric can be used in a single sheet or in a plurality of laminated sheets. In the latter case, the nonwoven fabrics are preferably bonded to each other with an adhesive or the like. In a case where a nonwoven fabric is used, it is desirable that the nonwoven fabric has a fiber basis weight of 10 to 50 g/m², particularly 15 to 30 g/m².

### (Intermediate sheet)

The intermediate sheet 15 is formed of a similar material to the top sheet 10. The intermediate sheet 15 is preferably bonded to the top sheet 10. In a case where heat embossing or ultrasonic welding is used for the bonding, a material of the intermediate sheet 15 preferably has approximately the same melting point as the top sheet 10. When a nonwoven fabric is used for the intermediate sheet 15, the fineness of fibers of the nonwoven fabric is preferably about 2.0 to 5.0 dtex.

### (Absorber)

The absorber 21 can be formed of an assembly of fibers. As this fiber assembly, in addition to those obtained by accumulating a short fiber such as fluff pulp or a synthetic fiber, a filament assembly obtained by opening a tow (fiber bundle) of a synthetic fiber such as cellulose acetate as necessary can also be used. In a case where fluff pulp or a short fiber is accumulated, a fiber basis weight may be, for example, about 100 to 300 g/m². In a case of a filament assembly, a fiber basis weight may be, for example, about 30 to 120 g/m². In a case of a synthetic fiber, a fineness is, for example, 1 to 16 dtex, preferably 1 to 10 dtex, and more preferably 1 to 5 dtex. In a case of a filament assembly, the filament may be formed of a non-crimped fiber but is preferably formed of a crimped fiber. The degree of crimp of the crimped fibers can be, for example, about 5 to 75, preferably 10 to 50, and more preferably 15 to 50 per inch. A uniformly crimped fiber is often used.

The absorber 21 preferably includes super absorbent polymer particles. In particular, it is desirable that the super absorbent polymer particles (SAP particles) are dispersed substantially in the entire thickness direction with respect to a fiber assembly at least in a liquid receiving region.

When there are no SAP particles in an upper portion, a lower portion, and an intermediate portion of the absorber 21, or when there are very few SAP particles therein, it cannot be said that the SAP particles are "dispersed in the entire thickness direction". Therefore, "dispersed in the entire thickness direction" includes not only a form in which the SAP particles are dispersed "uniformly" in the entire thickness direction with respect to a fiber assembly but also a form in which the SAP particles are "unevenly distributed" in the upper portion, the lower portion, and/or the intermediate portion but are still dispersed in each of the upper portion, the lower portion, and the intermediate portion. In addition, a form in which some of the SAP particles remain on a surface of the fiber assembly without entering the fiber assembly, and a form in which some of the SAP particles pass through the fiber assembly and are present on the wrapping sheet 22 are not excluded.

The super absorbent polymer particles include "powder" in addition to "particles". As the particle size of the super absorbent polymer particle, those used for this type of absorbent article can be used as they are, and it is desirable that the particle size is 1000 µm or less, particularly 150 to 400 µm. A material of the super absorbent polymer particles can be used without particular limitation, but those having a water absorption capacity of 40 g/g or more are preferable. Examples of the super absorbent polymer particles include a starch-based material, a cellulose-based material, and a synthetic polymer-based material. A starch-acrylic acid (salt) graft copolymer, a saponified product of a starch-acrylonitrile copolymer, a cross-linked product of sodium carboxymethyl cellulose, an acrylic acid (salt) polymer, or the like can be used. As the shapes of the super absorbent polymer particles, a usually used particulate material shape is suitable, but other shapes can also be used.

As the super absorbent polymer particles, those having an absorption speed of 70 seconds or less, particularly 40 seconds or less are suitably used. When the absorption speed is too slow, so-called returning that a liquid supplied into the absorber 21 returns out of the absorber 21 tends to occur.

The basis weight of the super absorbent polymer particles can be appropriately determined depending on the absorption amount required for an application of the absorber 21. Therefore, the basis weight can be 50 to 350 g/m² although this cannot be applied generally. The basis weight of the polymer of less than 50 g/m² makes it difficult to secure the absorption amount. When the basis weight exceeds 350 g/m², not only the effect is saturated but also the excess of the super absorbent polymer particles imparts a gritty and uncomfortable feeling.

### (Wrapping sheet)

The wrapping sheet 22 is formed of tissue paper, particularly crepe paper, a nonwoven fabric, a polylaminated nonwoven fabric, a sheet with small holes, or the like. However, it is desirable that the wrapping sheet 22 is a sheet from which super absorbent polymer particles do not escape. When a nonwoven fabric is used instead of crepe paper, a hydrophilic spunbonded/melt blown/melt blown/spunbonded (SMMS) nonwoven fabric is particularly suitable, and polypropylene, polyethylene/polypropylene, or the like can be used as a material thereof. A nonwoven fabric having a fiber basis weight of 5 to 40 g/m², particularly of 10 to 30 g/m² is desirable.

### (Three-dimensional gather)

A water repellent nonwoven fabric can be used as the gather sheet 31 of the three-dimensional gather 30, and a rubber thread or the like can be used as the elastically stretchable member 32. A plurality of elastically stretchable members can be disposed, or one elastically stretchable member can be disposed.

An opposite body-side surface of the gather sheet 31 has a fixed start point in the width direction on a side portion of the top sheet 10, and a portion outside in the width direction from the fixed start point is fixed to a side portion of the back sheet 11 and a side portion of the outer sheet 12 located outside the side portion of the back sheet 11 in the width direction with a hot melt adhesive or the like.

In a periphery of a leg, an inside of the fixed start point of the three-dimensional gather 30 in the width direction is fixed to the top sheet 10 at both ends in a product front-back direction. However, a portion therebetween is a non-fixed free portion, and the free portion rises by a contraction force of the elastically stretchable member 32. When the diaper is worn, the diaper is attached to a body in a boat shape, and therefore a contraction force of the elastically stretchable member 32 acts. Therefore, the three-dimensional gather 30 rises by the contraction force of the elastically stretchable member 32 and comes into close contact with a periphery of a leg. As a result, so-called side leakage from a periphery of a leg is prevented.

### (Planar gather)

The leg-surrounding elastically stretchable member 41 formed of rubber thread or the like is fixed in a state of being stretched in the front-back direction between the gather sheet 31 and the back sheet 11. A plurality of the elastically stretchable members 41 can be disposed, or one elastically stretchable member 41 can be disposed.

### (Fastening tape)

A base of the base material 51 of the fastening tape 50 is fixed between the gather sheet 31 and the outer sheet 12 with a hot melt adhesive or the like. The base material 51 is formed of a nonwoven fabric, a plastic film, a polylaminated nonwoven fabric, paper, or a composite material thereof.

The engagement portion 52 is formed of a hook material of a mechanical fastener. The hook member has a large number of engaging projections on an external surface side thereof. Examples of the shapes of the engaging projections include (A) tick shape, (B) J shape, (C) mushroom shape, (D) T shape, and (E) double J shape (a shape in which the J-shaped ones are connected to each other back to back), but any one of these shapes may be used. Of course, a pressure sensitive adhesive material layer can be disposed as the engagement portion of the fastening tape 50.

### (Target sheet)

The target sheet 55 is formed of, for example, a plastic film or a nonwoven fabric having many loop yarns on a surface thereof.

### (Stretchable sheet)

The stretchable sheet 60 is a stretchable sheet that stretches and contracts the end flap portion EF on the dorsal side to bring the end flap portion EF on the dorsal side into close contact with the back of a wearer. As illustrated in Fig. 4, the stretchable sheet 60 includes the inner sheet 61 formed of a nonwoven fabric, the outer sheet 62 formed of a nonwoven fabric, and the plurality of elongated stretchable members 63 disposed between the inner sheet 61 and the outer sheet 62 and extending in the width direction at a predetermined interval in the front-back direction. Note that the stretchable member 63 is disposed by stretching a rubber thread having a fineness of 470 to 620 dtex at a stretch rate of 200 to 250%.

Both side portions of the stretchable sheet 60 in the width direction are located in the vicinity of both side portions of the pair of left and right gather sheets, and both side portions of the stretchable member 63 in the width direction are located on both side portions of the back sheet 11.

### <Explanation of terms in specification>

In a case where the following terms are used in the specification, the terms have the following meanings unless otherwise specified in the specification.

- "Front-back (longitudinal) direction" means a direction connecting a ventral side (front side) and a dorsal side (dorsal side), and "width direction" means a direction orthogonal to the front-back direction (left-right direction).
- "Front surface side" means a side closer to the skin of a wearer. "Back surface side" means a side farther from the skin of a wearer. • "Front surface" means a surface closer to the skin of a wearer. "Back surface" means a surface farther from the skin of a wearer.
- "MD (Machine Direction) direction" and the "CD (Cross Direction) direction" mean a flow direction (MD direction) in a manufacturing facility and a lateral direction (CD direction) orthogonal thereto, respectively, one of which is a front-back direction of a product and the other is a width direction of the product. The MD direction of a nonwoven fabric is a direction of fiber orientation of the nonwoven fabric. The fiber orientation is a direction in which fibers of a nonwoven fabric are aligned, and can be determined **by,** for example, a measurement method in accordance with a fiber orientation test method based on zero distance tensile strength by the TAPPI standard method T471 or a simple measurement method for determining a fiber orientation direction based on a tensile strength ratio in a front-back direction and a width direction.

- "Unfolded" means a flatly unfolded state without contraction or slackness.
- "Stretch rate" means a value obtained when a natural length is 100%. For example, a stretch rate of 200% is synonymous with an elongation ratio of 2.
- "Gel strength" is measured as follows. To 49.0 g of artificial urine, 1.0 g of super absorbent polymer is added, and the resulting mixture is stirred with a stirrer. The gel thus generated is left in a thermohygrostat at 40°C × 60%RH for three hours. Thereafter, the temperature is returned to room temperature, and gel strength is measured with a curdmeter (Curdmeter-MAX ME-500 manufactured by I. Techno Engineering Co., Ltd.).
- "Artificial urine" is a mixture of 2% by weight of urea, 0.8% by weight of sodium chloride, 0.03% by weight of calcium chloride dihydrate, 0.08% by weight of magnesium sulfate heptahydrate, and 97.09% by weight of deionized water, and is used at a temperature of 40°C unless otherwise specified.
- "Basis weight" is measured as follows. A sample or a test piece is predried and then left in a test chamber or an apparatus in a standard state (test location is at a temperature of 23 ± 1°C and a relative humidity of 50 ± 2%) so as to have a constant weight. Predrying refers to causing a sample or a test piece to have a constant weight in an environment of a temperature of 100°C. Note that fibers having an official moisture regain of 0.0% do not have to be predried. A sample of 100 mm × 100 mm in size is cut out from a test piece having a constant weight using a template for sampling (100 mm × 100 mm). The weight of the sample is measured. The weight is multiplied by 100 to calculate the weight per square meter to be used as a basis weight.
- "Thickness" is automatically measured under conditions that a load is 0.098 N/cm² and a pressing area is 2 cm² using an automatic thickness meter (KES-G5 handy compression tester).
- "Water absorption capacity" is measured in accordance with JIS K7223-1996 "Test method for water absorption capacity of super absorbent polymer".
- "Absorption speed" is "time to end point" when JIS K7224-1996 "Test method for water absorption rate of super absorbent polymer" is performed using 2 g of super absorbent polymer and 50 g of physiological saline.
- In a case where environmental conditions in a test and a measurement are not described, the test and the measurement are performed in a test room or an apparatus in a standard state (test location is at a temperature of 23 ± 1°C and a relative humidity of 50 ± 2%).
- The size of each portion means a size not in a natural length state but in an unfolded state unless otherwise specified.

### Industrial Applicability

The present invention is applicable to an absorbent article such as a disposable diaper.

### Reference Signs List

- 10: Top sheet
- 10A: Ventral side portion
- 10B: Intermediate portion
- 10C: Dorsal side portion
- 11: Back sheet
- 21: Absorber
- 60: Stretchable sheet
- 61: Inner sheet
- 62: Outer sheet
- 63: Elastically stretchable member
- 70: Lotion portion
- 70A: Lotion portion
- EF: End flap portion

## Claims

1. A disposable diaper comprising:
an absorber (21) disposed between a liquid pervious top sheet (10) and a liquid impervious back sheet (11);
an end flap portion (EF) formed on each side of the absorber (21) in a front-back direction; and
a lotion portion (70) extending in the front-back direction at a predetermined interval in a width direction and formed on a body-side surface of the top sheet (10), wherein
in the front-back direction, the lotion portion (70) is disposed in an intermediate portion (10B) between a ventral side portion (10A) accounting for 20 to 45% of a front-back direction length of the top sheet (10) from a ventral side end of the top sheet (10) and a dorsal side portion (10C) accounting for 10 to 35% of the front-back direction length of the top sheet (10) from a dorsal side end of the top sheet (10), and
the lotion portion (70) is disposed in each of the ventral side portion (10A) and the dorsal side portion (10C), and a second application basis weight of a lotion agent forming the lotion portion (70) disposed in each of the ventral side portion (10A) and the dorsal side portion (10C) is smaller than a first application basis weight of the lotion agent forming the lotion portion (70) disposed in the intermediate portion (10B), the basis weight being measured as indicated in the description.

2. The disposable diaper according to claim 1, wherein the first application basis weight is 5 to 15 g/m², and the second application basis weight is 2 to 5 g/m², the basis weight being measured as indicated in the description.

3. The disposable diaper according to claim 1 or 2, wherein
a rectangular stretchable sheet (60) that stretches and contracts in the width direction is disposed between the top sheet (10) and the back sheet (11) forming the end flap portion (EF) on a dorsal side,
the stretchable sheet (60) is formed of an inner sheet (61) facing the top sheet (10), an outer sheet (62) facing the back sheet (11), and a stretchable member (63) that stretches and contracts in the width direction between the inner sheet (61) and the outer sheet (62) at a predetermined interval in the front-back direction,
the stretchable member (63) is fixed to an opposite body-side surface of the inner sheet (61) and a body-side surface of the outer sheet (62) via an adhesive portion (65) formed at a predetermined interval in the width direction, and
in plan view, a first interval of the lotion portion (70) in the width direction is wider than a second interval between the adhesive portion (65) and the adhesive portion (65) adjacent thereto in the width direction.

4. The disposable diaper according to any one of claims 1 to 3, wherein the lotion agent is water-soluble glycerin.

## Patentansprüche

1. Wegwerfwindel, umfassend:
einen Absorber (21), der zwischen einer flüssigkeitsdurchlässigen Decklage (10) und einer flüssigkeitsundurchlässigen hinteren Lage (11) vorgesehen ist;
einen Endklappenabschnitt (EF), der auf jeder Seite des Absorbers (21) in einer Vorderseiten-Rückseiten-Richtung ausgebildet ist; und
einen Lotionabschnitt (70), der sich in der Vorderseiten-Rückseiten-Richtung in einem vorbestimmten Abstand in einer Breitenrichtung erstreckt und auf einer körperseitigen Oberfläche der Decklage (10) ausgebildet ist, wobei der Lotionabschnitt (70) in der Vorderseiten-Rückseiten-Richtung in einem Zwischenabschnitt (10B) zwischen einem bauchseitigen Abschnitt (10A), der 20 bis 45 % einer Länge der Vorderseiten-Rückseiten-Richtung der Decklage (10) von einem bauchseitigen Ende der Decklage (10) ausmacht, und einem rückenseitigen Abschnitt (10C), der 10 bis 35% der Länge der Vorderseiten-Rückseiten-Richtung der Decklage (10) von einem rückenseitigen Ende der Decklage (10) ausmacht, vorgesehen ist, und
der Lotionabschnitt (70) in jedem des bauchseitigen Abschnitts (10A) und des rückenseitigen Abschnitts (10C) vorgesehen ist, und ein zweites Auftragsflächengewicht eines Lotionwirkstoffs, der den Lotionabschnitt (70) bildet, der in jedem des bauchseitigen Abschnitts (10A) und des rückenseitigen Abschnitts (10C) vorgesehen ist, kleiner ist als ein erstes Auftragsflächengewicht des Lotionwirkstoffs, der den Lotionabschnitt (70) bildet, der im Zwischenabschnitt (10B) vorgesehen ist, wobei das Flächengewicht wie in der Beschreibung angegeben gemessen wird.

2. Wegwerfwindel nach Anspruch 1, wobei das erste Auftragsflächengewicht 5 bis 15 g/m² beträgt und das zweite Auftragsflächengewicht 2 bis 5 g/m² beträgt, wobei das Flächengewicht wie in der Beschreibung angegeben gemessen wird.

3. Wegwerfwindel nach Anspruch 1 oder 2, wobei
eine rechteckige, dehnbare Lage (60), die sich in der Breitenrichtung dehnt und zusammenzieht, zwischen der Decklage (10) und der hinteren Lage (11) vorgesehen ist und den Endklappenabschnitt (EF) auf einer Rückenseite bildet,
die dehnbare Lage (60) aus einer zur Decklage (10) gewandten inneren Lage (61), einer zur hinteren Lage (11) gewandten äußeren Lage (62), und einem dehnbaren Element (63), das sich zwischen der inneren Lage (61) und der äußeren Lage (62) in Breitenrichtung dehnt und zusammenzieht, in einem vorbestimmten Abstand in der Vorderseiten-Rückseiten-Richtung gebildet ist,
das dehnbare Element (63) an einer gegenüberliegenden körperseitigen Oberfläche der inneren Lage (61) und einer körperseitigen Oberfläche der äußeren Lage (62) über einen Klebeabschnitt (65) befestigt ist, der in einem vorbestimmten Abstand in der Breitenrichtung gebildet ist, und
in der Draufsicht ein erster Abstand des Lotionabschnitts (70) in der Breitenrichtung breiter ist als ein zweiter Abstand zwischen dem Klebeabschnitt (65) und dem in Breitenrichtung dazu benachbarten Klebeabschnitt (65) ist.

4. Wegwerfwindel nach einem der Ansprüche 1 bis 3, wobei der Lotionwirkstoff wasserlösliches Glycerin ist.

## Revendications

1. Couche jetable comprenant :
un absorbeur (21) disposé entre une feuille supérieure perméable aux liquides (10) et une feuille arrière imperméable aux liquides (11) ;
une partie rabat d'extrémité (EF) formée de chaque côté de l'absorbeur (21) dans une direction avant-arrière ; et
une partie lotion (70) s'étendant dans la direction avant-arrière à un intervalle prédéterminé dans un sens de la largeur et formée sur une surface côté corps de la feuille supérieure (10), dans laquelle
en direction avant-arrière, la partie lotion (70) est disposée dans une partie intermédiaire (10B) entre une partie côté ventral (10A) représentant 20 à 45 % d'une longueur en direction avant-arrière de la feuille supérieure (10) à partir d'une extrémité côté ventral de la feuille supérieure (10) et une partie côté dorsal (10C) représentant 10 à 35 % de la longueur en direction avant-arrière de la feuille supérieure (10) à partir d'une extrémité côté dorsal de la feuille supérieure (10), et la partie lotion (70) est disposée dans chacune de la partie côté ventral (10A) et de la partie côté dorsal (10C), et un second poids de base d'application d'un produit de type lotion formant la partie lotion (70) disposée dans chacune de la partie côté ventral (10A) et de la partie côté dorsal (10C) est inférieur à un premier poids de base d'application du produit de type lotion formant la partie lotion (70) disposée sur la partie intermédiaire (10B), le poids de base étant mesuré comme indiqué dans la description.

2. Couche jetable selon la revendication 1, dans laquelle le premier poids de base d'application est de 5 à 15 g/m², et le second poids de base d'application est de 2 à 5 g/m², le poids de base étant mesuré comme indiqué dans la description.

3. Couche jetable selon la revendication 1 ou 2, dans laquelle
une feuille rectangulaire extensible (60) qui s'étire et se contracte dans le sens de la largeur est disposée entre la feuille supérieure (10) et la feuille arrière (11) formant la partie rabat d'extrémité (EF) sur un côté dorsal, la feuille extensible (60) est formée d'une feuille interne (61) faisant face à la feuille supérieure (10), d'une feuille externe (62) faisant face à la feuille arrière (11) et d'un organe extensible (63) qui s'étire et se contracte dans le sens de la largeur entre la feuille interne (61) et la feuille externe (62) à un intervalle prédéterminé dans la direction avant-arrière,
l'organe extensible (63) est fixé à une surface côté corps opposée de la feuille interne (61) et à une surface côté corps de la feuille externe (62) par l'intermédiaire d'une partie adhésive (65) formée à un intervalle prédéterminé dans le sens de la largeur, et
en vue en plan, un premier intervalle de la partie lotion (70) dans le sens de la largeur est plus large qu'un second intervalle entre la partie adhésive (65) et la partie adhésive (65) adjacente à celle-ci dans le sens de la largeur.

4. Couche jetable selon l'une quelconque des revendications 1 à 3, dans laquelle le produit de type lotion est de la glycérine hydrosoluble.
